**Europäisches Patentamt**

**European Patent Office**

**Office européen des brevets**

(19)

(11) Publication number : **0 403 282 B1**

(12) # EUROPEAN PATENT SPECIFICATION

(45) Date of publication of patent specification :
**02.02.94 Bulletin 94/05**

(51) Int. Cl.⁵ : **A61K 7/06, A61K 7/11**

(21) Application number : **90306499.6**

(22) Date of filing : **14.06.90**

(54) Hair setting composition.

The file contains technical information
submitted after the application was filed and
not included in this specification

(30) Priority : **15.06.89 GB 8913821**

(43) Date of publication of application :
**19.12.90 Bulletin 90/51**

(45) Publication of the grant of the patent :
**02.02.94 Bulletin 94/05**

(84) Designated Contracting States :
**AT BE CH DE DK ES FR GB GR IT LI NL SE**

(56) References cited :
**WO-A-90/14071**
**FR-A- 2 354 760**
**GB-A- 2 026 516**
**PATENT ABSTRACTS OF JAPAN vol. 8, no. 30
(C-209)(1467) 8 February 1984, & JP-A-58
192816 (KIKKOMAN SHOYU K.K.) 10 Novem-
ber 1983,**

(73) Proprietor : **UNILEVER PLC**
**Unilever House Blackfriars**
**London EC4P 4BQ (GB)**

(84) **GB**
Proprietor : **UNILEVER N.V.**
**Weena 455**
**NL-3013 AL Rotterdam (NL)**

(84) **BE CH DE DK ES FR GR IT LI NL SE AT**

(72) Inventor : **McGee, Thomas**
**74 Stanley Road, Hoylake**
**Wirral, Merseyside L47 1H7 (GB)**
Inventor : **Rossall, Brian**
**11 Redmere Drive, Heswall**
**Wirral, Merseyside L60 1YF (GB)**
Inventor : **Gallagher, Peter**
**1 Mission Cottage (off Moss Lane)**
**Burscough, Lancashire (GB)**

(74) Representative : **Ford, Michael Frederick et al**
**MEWBURN ELLIS 2 Cursitor Street**
**London EC4A 1BQ (GB)**

## Description

The present invention relates to a hair setting composition, and more particularly to a hair setting composition containing water soluble salts of a natural polymer complex which gives superior curl retention.

The use of hair setting preparations for holding hair in place and giving style retention is well known. The setting agent acts by forming a film on the hair filaments, holding them together sufficiently to retain the hair in the required style for some hours.

The setting agents used in these preparations can include film-forming synthetic polymers or natural materials. Suitable synthetic polymers include polyvinylpyrrolidone, polyvinyl acetate, poly(acrylic acid), poly(methacrylic acid), polyacrylonitrile, a copolymer of vinyl acetate and butonic acid or a copolymer of methyl vinyl ether and maleic anhydride.

Natural materials which form stable films may also be used in such preparations and examples of suitable materials include Shellac, Irish moss, alginates, gelatines, pectins, cellulose derivatives and gum tragacanth.

Hair setting preparations containing water soluble salts of chitosan are disclosed in GB 1 583 086 and its equivalent FR-A-23 54 760 (Wella). Chitosan is a deactylation product of chitin, a natural polyglucosamine whose amine groups are acetylated, and which is found in the shells of crustacea, and in certain insects. In other species chitin occurs in a complex with glucan.

It is a common problem that, in climates having high humidity, or in humid atmospheres, the films formed by conventional setting agents absorb water and lose their ability to maintain a set. Attempts have been made to improve set retention by the addition of hydrophobic materials such as isopropyl myristate or dimethyl phthalate to the polymer, or by careful selection of synthetic polymers to achieve a more hydrophobic film.

## BRIEF SUMMARY OF THE INVENTION

It has now been found that superior style retention at high humidity can be achieved by using a particular polymer, without the need for added hydrophobic materials.

Accordingly, the invention provides a setting composition for setting hair comprising water soluble salts of polyaminoglucose glycan polymer complex, the composition having a pH of less than 7.

## DETAILED DESCRIPTION OF THE INVENTION

The film-forming material used in the setting composition of the invention is a water-soluble salt of a complex between polyaminoglucose and glycan polymer.

Such a polymer complex useful for this invention may be obtained by extraction from the mycelium of <u>Aspergillus niger</u>. <u>Aspergillus niger</u> is a common waste product in industrial fermentation processes, for example in the production of citric acid.

A complex of polyaminoglucose and glycan, as used in this invention has not been fully characterised, but NMR studies have shown that it is distinct in character from chitosan, and physical comparisons show that films formed by such a complex and by chitosan are different from each other.

A polyaminoglucose glycan polymer complex which can be used in the compositions of the invention, is commercially available, for example as RIOSAN (Trademark) from Meyhall.

The water soluble salts are obtained by treating the polyaminoglucose glycan polymer complex with a suitable acid. Sufficient acid is added to lower the pH to below 7, preferably to a pH of from 2 to 6.5, ideally to a pH of 4. Suitable acids include hydrochloric, lactic, acetic, formic, malonic, glycolic, thioglycollic. benzoic, adipic, malic, mesaconic, citric, benzodisulphonic and chlorosulphonic acids. Preferred acids are formic, malic, lactic and mesaconic acids.

The hair setting compositions according to the invention suitable contain from 0.05 to 20% by weight of the water soluble salt of polyaminoglucose glycan polymer complex, preferably from 0.1 to 5% by weight.

The salts of polyaminoglucose glycan polymer complex are suitably dissolved in an aqueous or an aqueous/alcoholic base. Suitable alcohols include the lower ($C_{1-4}$) alcohols such as ethanol and isopropanol.

In addition to the water soluble salts of polyaminoglucose glycan polymer complex, the setting compositions of the invention may comprise a conditioning agent, for example in an amount of from 0.01 to 10 % by weight. The conditioning agent is preferably chosen from cationic surfactants, cationic polymers, quaternised silicones, volatile silicones, quaternized polymers, protein hydrolysates or quaternised protein hydrolysates.

Examples of cationic surfactants include
Cetyl trimethylammonium chloride
Stearyl dimethylbenzyl ammonium chloride

Cetylpyridinium chloride

Quaternium -5

Quaternium -31

Quaternium -18

and mixtures thereof

Suitable cationic polymers include

Guar Hydroxypropyltrimonium chloride

Quaternium -19

Quaternium -23

Quaternium -40

Quaternium -57

Poly(dimethyldiallyammonium chloride)

Poly(dimethylbutenyl ammonium chloride)-$\alpha,\omega$-bis(triethanolammonium chloride)

Poly(dipropyldiallylammonium chloride)

Poly(methyl-$\beta$-propaniodiallyammonium chloride)

Poly(diallylpiperidinium chloride)

Poly(vinyl pyridinium chloride)

Quaternised poly (vinyl alcohol)

Quaternised poly (dimethylaminoethylmethacrylate) and mixtures thereof.

Examples of suitable volatile silicone materials include cyclodimethicone, available commercially as Dow Corning DC 345, and Volatile Silicone 7158, available from Union Carbide.

The quaternised silicones which may be used in the compositions of the invention are generally amino functional polydimethylsiloxanes. Examples include amodimethicone available commercially as Dow Corning DC 929 and trimethylsilylamodimethicone, available as Dow Corning Q 8220.

Suitable protein derivatives include lauryl dimonium hydroxy propylamino hydrolysed animal protein, available commercially under the trade name LAMEQUAT L, and hydrolysed keratin containing sulphur-bearing amino acids, available commercially under the trade name CROQUAT WKP.

The compositions of the invention may also comprise conventional ingredients such as surfactant, perfume, colour, emollient, foam stabiliser, UV absorber, preservative, thickener, or suspending agent.

The hair setting compositions according to the invention may for example, be used in styling, setting, shaping or blow drying products, and may take the form of lotions, gels, mousses or sprays. These products are designed to be used on washed, wet hair before styling, or on dry hair to restyle or to retain the shape of the style.

When the compositions take the form of pressurized compositions in aerosols to form sprays or mousses, they additionally comprise a propellant, for example carbon dioxide, nitrogen, nitrous oxide, a volatile hydrocarbon such as butane, isobutane or propane, or a chlorofluorocarbon.

The following Comparative Examples illustrate the benefits of the invention.

COMPARATIVE EXAMPLES

Comparative Example 1

Test of film forming ability

0.2g of the polyaminoglucose glycan polymer complex was suspended in 18g of distilled water. 0.2g of acid was added, and the mixture was stirred for 30 minutes. Further aliquots of acid were added as necessary with stirring, and optional heating, until complete dissolution was obtained. The weight of the solution was then made up to 20g with distilled water. The solution was then poured into a Petri dish and allowed to evaporate overnight at 40-50°C. The resulting film was then examined and assessed for quality. Results are shown below.

| Acid | Film quality |
|------|--------------|
| Hydrochloric | Opaque/crystalline |
| Acetic | Clear/coherent |
| Formic | Clear/coheren* |
| Citric | Clear/tacky |
| Adipic | Clear/coherent |
| Mesaconic | Clear/coherent |
| Malic | Clear/coherent |
| Benzoic * | Clear/crystalline |

\* Required heating to obtain dissolution

The polymer complex formed films with all acids tested, and it can be seen that acetic, formic, adipic, mesaconic and malic acids give clear, coherent films.

Comparative Example 2

Comparison of curl retention

The property of curl retention can be used to compare the performance of known hair setting compositions with that of the hair setting composition according to the invention.

Curl retention is defined as

$$CR = \frac{L_\infty - L_t}{L_\infty} \times 100$$

where

$L_\infty$ is the extended length of the hair switch

$L_t$ is the length of the curl at time t

The curl retention given by hair setting compositions containing, as hair setting agent

(a) the acetic acid salt of chitosan,

(b) polyvinyl pyrrolidone/vinyl acetate copolymer, and

(c) the acetic acid salt of polyaminoglucose glycan polymer complex,

were measured as follows.

Three 20cm Yugoslavian virgin hair switches each weighing 1g were first shampooed using a 1.6% by weight aqueous solution of sodium lauryl ether sulphate, and rinsed under running water. To each switch, was added 0.05g of a 1% by weight solution of the setting agent to be tested. The hair switch was then wound on a 1cm polytetrafluoroethylene roller and dried at 50°C for 1 hour. The roller and hair switch were equilibrated at 85% relative humidity overnight.

The switch was then removed from the roller and hung up in a constant humidity chamber at 85% relative humidity and the length measured at predetermined time intervals. The results are as shown below in Table 1.

## Table 1

| Time (mins) | CR | | |
| --- | --- | --- | --- |
| | (a) | (b) | (c) |
| 5 | 56 | 56 | 58 |
| 30 | 54 | 52 | 56 |
| 60 | 52 | 49 | 55 |
| 90 | 51 | 47.3 | 54.1 |
| 180 | 49 | 43.8 | 52.6 |

It can be seen from the results in Table 1 that for setting agents (a) and (b), the curl retention decreases markedly in an atmosphere of high humidity. Setting agent (c) the polyaminoglucose glycan polymer complex used in the composition of the invention, demonstrates good curl retention even after 180 minutes in an atmosphere of 85% relative humidity.

EXAMPLES

The invention is further illustrated by the following examples. Each example uses polyaminoglucose glycan polymer complex and an acid. These form water-soluble salts of the complex in the setting compositions. The pH of each composition is less than 7. In each example the polyaminoglucose glycan polymer complex is that sold under the Trade Mark RIOSAN.

Example 1

Hair setting mousse

| | % wt |
| --- | --- |
| Riosan (Polyaminoglucose glycan polymer complex) | 3.3 |
| Glacial acetic acid | 3.3 |
| Lauryl amine oxide | 0.01 |
| F12 (propellant) | 15.0 |
| Perfume | qs |
| Water | to 100 |

### Example 2

**Hair setting mousse**

|                      | % wt   |
|----------------------|--------|
| Riosan               | 1.7    |
| Glacial acetic acid  | 1.7    |
| Lauryl amine oxide   | 0.01   |
| CAP 30 (propellant)  | 7.0    |
| Perfume              | qs     |
| Water                | to 100 |

### Example 3

**Hair setting mousse**

|                      | % wt   |
|----------------------|--------|
| Riosan               | 2.6    |
| Formic acid          | 2.6    |
| Lauryl amine oxide   | 0.01   |
| CAP 30 (propellant)  | 7.0    |
| Ethanol              | 15.0   |
| Perfume              | qs     |
| Water                | to 100 |

### Example 4

**Hair setting lotion**

|             | % wt   |
|-------------|--------|
| Riosan      | 4.0    |
| Acetic acid | 4.0    |
| TWEEN 20*   | 0.05   |
| Ethanol     | 20     |
| Perfume     | qs     |
| Water       | to 100 |

* TWEEN 20 (Trade Mark) has the CTFA designation polysorbate 20 and is sorbitan monolaurate condensed with average 20 moles of ethylene oxide.

Example 5

Hair setting lotion

| | % wt |
|---|---|
| Riosan | 0.2 |
| Formic acid | 0.2 |
| TWEEN 20 | 0.05 |
| Ethanol | 25 |
| Perfume | qs |
| Water | to 100 |

**Claims**

1.  A setting composition for setting hair comprising water soluble salts of polyaminoglucose glycan polymer complex, the composition having a pH of less than 7.

2.  A setting composition as claimed in Claim 1 wherein the polyaminoglucose glycan polymer complex is obtained by extraction from Aspergillus niger.

3.  A setting composition as claimed in any preceding claim wherein the water soluble salt of polyaminoglucose glycan polymer complex is present in an amount of from 0.05 to 20% by weight in an aqueous composition.

4.  A setting composition as claimed in claim 3 wherein the water soluble salt of polyaminoglucose glycan polymer complex is present in an amount of from 0.1 to 5% by weight.

5.  A setting composition as claimed in any preceding claim wherein the water soluble salts of the polyaminoglucose glycan polymer complex are the salts of hydrochloric acid, lactic acid, acetic acid, formic acid, malonic acid, glycolic acid, thioglycolic acid, benzoic acid, adipic acid, malic acid or mesaconic acid.

6.  A setting composition as claimed in Claim 5 wherein the water soluble salts of the polyaminoglucose glycan polymer complex are the salts of formic acid, malic acid, lactic acid or mesaconic acid.

7.  A setting composition as claimed in any preceding claim wherein the pH of the composition is from 2 to 6.5.

8.  A setting composition as claimed in Claim 7 wherein the pH of the composition is 4.

9.  A setting composition as claimed in any preceding claim wherein the composition further comprises from 0.01 to 10% by weight of a conditioning agent.

10. A setting composition as claimed in claim 9 wherein the conditioning agent is chosen from cationic surfactants, cationic polymers, quaternised silicones, volatile silicones, quaternised polymers, protein hydrolysates or quaternised protein hydrolysates.

**Patentansprüche**

1.  Fixierungszusammensetzung zum Fixieren von Haar umfassend wasserlösliche Salze des Polyaminoglucose-Glykanpolymerkomplexes, wobei die Zusammensetzung einen pH-Wert von weniger als 7 aufweist.

2. Fixierungszusammensetzung gemäß Anspruch 1, in der der Polyaminoglucose-Glykanpolymerkomplex durch Extraktion aus Aspergillus niger erhalten ist.

3. Fixierungszusammensetzung gemäß einem der vorangehenden Ansprüche, in der das wasserlösliche Salz des Polyaminoglucose-Glykanpolymerkomplexes in einer Menge von 0,05 bis 20 Gewicht-% in einer wäßrigen Zusammensetzung vorhanden ist.

4. Fixierungszusammensetzung gemäß Anspruch 3, in der das wasserlösliche Salz des Polyaminoglucose-Glykanpolymerkomplexes in einer Menge von 0,1 bis 5 Gewicht-% vorhanden ist.

5. Fixierungszusammensetzung gemäß einem der vorangehenden Ansprüche, in der die wasserlöslichen Salze des Polyaminoglucose-Glykanpolymerkomplexes die Salze der Salzsäure, Milchsäure, Essigsäure, Ameisensäure, Malonsäure, Glycolsäure, Thioglycolsäure, Benzoesäure, Adipinsäure, Apfelsäure oder Mesaconsäure sind.

6. Fixierungszusammensetzung gemäß Anspruch 5, in der die wasserlöslichen Salze des Polyaminoglucose-Glykanpolymerkomplexes die Salze der Ameisensäure, Apfelsäure, Milchsäure oder Mesaconsäure sind.

7. Fixierungszusammensetzung gemäß einem der vorangehenden Ansprüche, wobei der pH-Wert der Zusammensetzung von 2 bis 6,5 beträgt.

8. Fixierungszusammensetzung gemäß Anspruch 7, wobei der pH-Wert der Zusammensetzung 4 beträgt.

9. Fixierungszusammensetzung gemäß einem der vorangehenden Ansprüche, wobei die Zusammensetzung weiterhin von 0,01 bis 10 Gewicht-% eines Konditioniermittels umfaßt.

10. Fixierungszusammensetzung gemäß Anspruch 9, in der das Konditioniermittel ausgewählt ist unter kationischen Tensiden, kationischen Polymeren, quaternisierten Silikonen, flüchtigen Silikonen, quaternisierten Polymeren, Proteinhydrolysaten oder quaternisierten Proteinhydrolysaten.


**Revendications**

1. Une composition de fixation pour fixer la chevelure comprenant des sels solubles dans l'eau d'un complexe polymère de polyaminoglucose et de glycane, la composition ayant un pH inférieur à 7.

2. Une composition de fixation selon la Revendication 1, dans laquelle le complexe polymère de polyaminoglucose et de glycane est obtenu par extraction à partir de Aspergillus niger.

3. Une composition de fixation selon l'une des revendications précédentes, dans laquelle le sel soluble dans l'eau de complexe polymère de polyaminoglucose et de glycane est présent dans une quantité allant de 0,05 à 20% en masse dans une composition aqueuse.

4. Une composition de fixation selon la revendication 3, dans laquelle le sel soluble dans l'eau de complexe polymère de polyaminoglucose et de glycane est présent dans une quantité allant de 0,1 à 5% en masse.

5. Une composition de fixation selon l'une des revendications précédentes, dans laquelle les sels solubles dans l'eau de complexe polymère de polyaminoglucose et de glycane sont les sels d'acide chlorhydrique, d'acide lactique, d'acide acétique, d'acide formique, d'acide malonique, d'acide glycolique, d'acide thioglycolique, d'acide benzoïque, d'acide adipique, d'acide malique ou d'acide mésaconique.

6. Une composition de fixation selon la revendication 5, dans laquelle les sels solubles dans l'eau de complexe polymère de polyaminoglucose et de glycane sont les sels d'acide formique, d'acide malique, d'acide lactique ou d'acide mésaconique.

7. Une composition de fixation selon l'une des revendications précédentes, dans laquelle le pH de la composition va de 2 à 6,5.

8. Une composition de fixation selon la revendication 7, dans laquelle le pH de la composition est de 4.

9. Une composition de fixation selon l'une des revendications précédentes, dans laquelle la composition comprend en outre de 0,01 à 10% en masse d'une agent de conditionnement.

10. Une composition de fixation selon la revendication 9, dans laquelle l'agent de conditionnement est choisi à partir d'agents tensioactifs cationiques, de polymères cationiques, de silicones quaternisées, de silicones volatiles, de polymères quaternisés, d'hydrolysats de protéine ou d'hydrolysats de protéine quaternisée.